# EUROPEAN PATENT SPECIFICATION

(11) **EP 4 223 293 B1**
(45) Date of publication and mention of the grant of the patent: **27.05.2026**
(21) Application number: 21874791.3
(22) Date of filing: 20.04.2021
(51) Int. Cl.: A61K 31/37, A61P 35/00, A61K 31/404, A61K 31/405, A61K 31/453, A61K 31/5383, A61P 13/08, A61P 15/00, A61K 31/352, A61K 31/5365

(54) **PHARMACEUTICAL COMPOSITION FOR TREATMENT OF PROSTATE CANCER**
PHARMAZEUTISCHE ZUSAMMENSETZUNG ZUR BEHANDLUNG VON PROSTATAKREBS
COMPOSITION PHARMACEUTIQUE POUR LE TRAITEMENT DU CANCER DE LA PROSTATE

(30) Priority: 30.09.2020 JP 2020165745
(43) Date of publication of application: 09.08.2023
(73) Proprietor: Tokyo Metropolitan Geriatric Hospital and Institute of Gerontology, Tokyo 173-0015 (JP)
(72) Inventor: INOUE Satoshi, Tokyo 173-0015 (JP); TAKAYAMA Ken-ichi, Tokyo 173-0015 (JP); OSADA Hiroyuki, Wako-shi, Saitama 351-0198 (JP); KONDOH Yasumitsu, Wako-shi, Saitama 351-0198 (JP)
(74) Representative: Gille Hrabal Partnerschaftsgesellschaft mbB Patentanwälte
(86) International application number: PCT/JP2021/015982
(87) International publication number: WO 2022/070483

(56) References cited:
- WO-A2-2007/112051
- JP-A- 2007 536 226
- JP-A- 2009 062 300
- JP-A- H09 510 689
- US-A1- 2003 087 946
- US-B2- 8 853 182
- HARADA K ET AL: "Coumarins as novel 17^2-hydroxysteroid dehydrogenase type 3 inhibitors for potential treatment of prostate cancer", BIOORGANIC & MEDICINAL CHEMISTRY LETTERS, ELSEVIER, AMSTERDAM NL, vol. 20, no. 1, 1 January 2010 (2010-01-01), pages 272 - 275, XP026808821, ISSN: 0960-894X, [retrieved on 20091110]
- KEN-ICHI TAKAYAMA ET AL: "Dysregulation of spliceosome gene expression in advanced prostate cancer by RNA-binding protein PSF", PROCEEDINGS OF THE NATIONAL ACADEMY OF SCIENCES, vol. 114, no. 39, 11 September 2017 (2017-09-11), pages 10461 - 10466, XP055763981, ISSN: 0027-8424, DOI: 10.1073/pnas.1706076114
- TAKAYAMA KEN-ICHI ET AL: "Targeting Epigenetic and Posttranscriptional Gene Regulation by PSF Impairs Hormone Therapy-Refractory Cancer Growth", CANCER RESEARCH, vol. 81, no. 13, 11 May 2021 (2021-05-11), pages 3495 - 3508, XP093349607, ISSN: 0008-5472, DOI: 10.1158/0008-5472.CAN-20-3819
- ZHANG KEYUN; DING WEIXIAN; SUN JIE; ZHANG BIN; LU FUJIAO; LAI REN; ZOU YONG; YEDID GABRIEL: "Antioxidant and antitumor activities of 4-arylcoumarins and 4-aryl-3,4-dihydrocoumarins", BIOCHIMIE, MASSON, PARIS, FR, vol. 107, 8 April 2014 (2014-04-08), FR , pages 203 - 210, XP029103927, ISSN: 0300-9084, DOI: 10.1016/j.biochi.2014.03.014
- YANG, L. ET AL.: "Dual functional small molecule fluorescent probes for image-guided estrogen receptor-specific targeting coupled potent antiproliferative potency for breast cancer therapy", BIOORGANIC & MEDICINAL CHEMISTRY, vol. 25, 2017, pages 3531 - 3539, XP085037271, DOI: 10.1016/j.bmc.2017.05.002

## Description

### TECHNICAL FIELD

The present invention relates to a pharmaceutical composition for use in treating prostate cancer. According to the present invention, refractory prostate cancer can be treated

The invention is defined in the claims. Any subject-matter which is not encompassed by the claims is provided for reference or comparison only.

Any reference to a method of treatment of the human or animal body by therapy using a certain compound or composition is to be understood as relating to said compound or composition for use in said method of treatment.

### BACKGROUND ART

Prostate cancer and breast cancer are the most common cancers affecting men and women in Europe and the United States. In Japan, the number of patients with prostate cancer is increasing dramatically due to the Westernisation of the diet and the ageing of the population. The growth of prostate cancer is generally stimulated by androgen, which is a male hormone. On the other hand, estrogen, which is a female hormone, stimulates the growth of breast cancer. Therefore, hormone therapies that inhibit androgen and estrogen production and function are usually used for the treatment of prostate cancer and breast cancer. Although the effect of hormone therapy is initially excellent, prostate cancer relapses within a few years as castration-resistant prostate cancer (CRPC). In addition, the acquisition of resistance to hormone therapy is also a problem in breast cancer (PATENT LITERATURES 1 and 2). Thus, the control of treatment-resistant cancers that are resistant to hormone therapy is the most important problem to be solved.

### CITATION LIST

### PATENT LITERATURE

[PATENT LITERATURE 1] WO 2014/188775
[PATENT LITERATURE 2] JP 2013-17414 A
HARADA K ET AL: (BIOORGANIC & MEDICINAL CHEMISTRY LETTERS, ELSEVIER, AMSTERDAM NL, vol. 20, no. 1, 1 January 2010 (2010-01-01), pages 272-275) disclose coumarins as novel 17beta-hydroxysteroid dehydrogenase type 3 inhibitors for a potential treatment of prostate cancer.

### SUMMARY OF INVENTION

### TECHNICAL PROBLEM

The object of the present invention is to provide a therapeutic agent for use in treating hormone therapy-resistant, treatment-resistant prostate cancers.

### SOLUTION TO PROBLEM

The inventors have studied intensively on therapeutic agents and methods for treating hormone therapy-resistant, treatment-resistant cancers. Surprisingly, they found that compounds with specific chemical structures can effectively treat hormone therapy-resistant, treatment-resistant prostate cancers.

The present invention is based on these findings.

Therefore, the present invention relates to a pharmaceutical composition for use in treating prostate cancer comprising a compound selected from the group consisting of

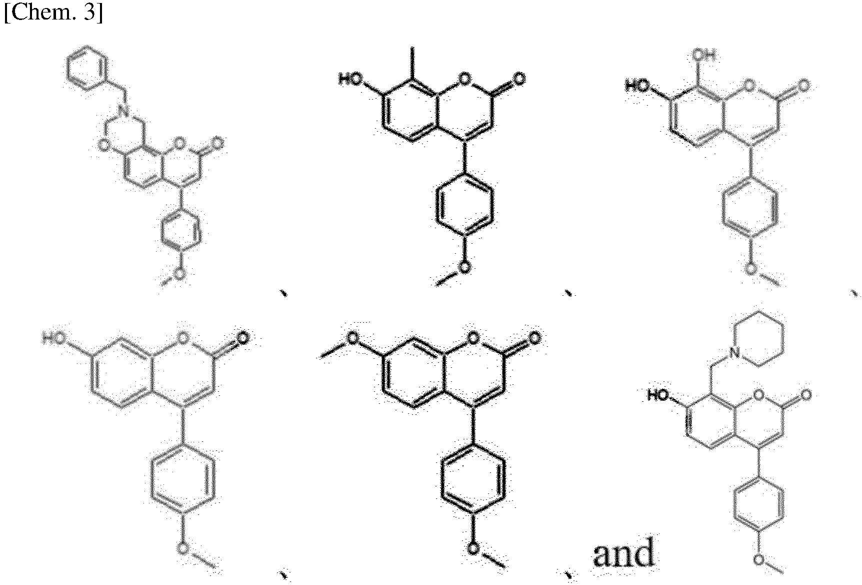

### ADVANTAGEOUS EFFECTS OF INVENTION

According to the pharmaceutical composition of the present invention, hormone therapy-resistant prostate cancer can be effectively treated. Furthermore, the pharmaceutical composition of the present invention can effectively treat not only hormone therapy-resistant prostate cancer but also non-hormone therapy-resistant prostate and Castration-resistant prostate cancer.

### BRIEF DESCRIPTION OF THE DRAWINGS

FIG. 1 is a graph showing the proliferation inhibitory effects of compounds 10-0, 10-1, 10-3, 14-0, and 14-5 on hormone therapy-resistant prostate cancer model cell line 22Rv1, hormone therapy-resistant model LTAD (long time androgen deprived) cells, and VCaP cells.
FIG. 2 is a graph showing the result of the measurement of the inhibitory capacity of compounds 10-0, 10-1, 10-2, 10-3, 10-4, 14-0, and 14-5 to PSF interaction with its target RNA by the RNA pull-down method.
FIG. 3 is a graph showing the cell proliferation inhibitory effects of compounds 10-0, 10-1, 10-3, 10-4, 14-0, and 14-5 using 22Rv1 cells (prostate cancer), MCF7 cells (breast cancer), and OHTR cells (breast cancer).
FIG. 4 is a graph showing the inhibition of expression of the target genes of PSF, which are AR, AR-V7, SchLaP1, FKBP5, and ACSL3, in 22Rv1 cells (prostate cancer), by compounds 10, 10-1, and 10-3.
FIG. 5 is a graph showing the inhibition of binding of compounds 10, 10-1, and 10-3 to the mRNAs of ERα, SCFD2, and TRA2B, which are the target genes of PSF, and GAPDH (control), in OHT-TamR cells (breast cancer).
FIG. 6 is a diagram showing the results of examining the in vivo anticancer effects of compound 10-3 using a mouse model in which AR-positive CRPC tumors (22Rv1 cells) are subcutaneously transplanted.
FIG. 7 is a graph and photographs showing the results of examining the expression of Ki67 and AR in tumor tissues treated with compound 10-3.
FIG. 8 is a graph showing the cell proliferation inhibitory effects of compounds 10-1 and 10-3 using a model cell (DU145 cells) of Castration-resistant prostate cancer, which is AR-negative and has a mutation of tumor suppressor p53.
FIG. 9 is a diagram showing the in vivo anticancer effects of compound 10-3 using a mouse model in which AR-negative CRPC tumors (DU145 cells) are subcutaneously transplanted.
FIG. 10 is a diagram showing the results of examining the binding of the three PSF mutants (mut#1:K516I, D517V, mut#2:Y490H, mut#1+2:Y490H, K516I, D517V) and wild-type PSF to the RNA strand of CTBP1-AS, confirmed by RNA pull-down method. Docking analysis to determine the binding site of compound 10-3 to its target protein, PSF, suggested that the position at which each mutation was introduced interacted with compound 10-3.

### DESCRIPTION OF EMBODIMENTS

The pharmaceutical composition for use in treating prostate cancer of the present invention comprises: a compound (hereinafter referred to as compound A) represented by the following formulae (10-0) to (10-5):

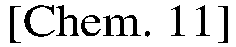

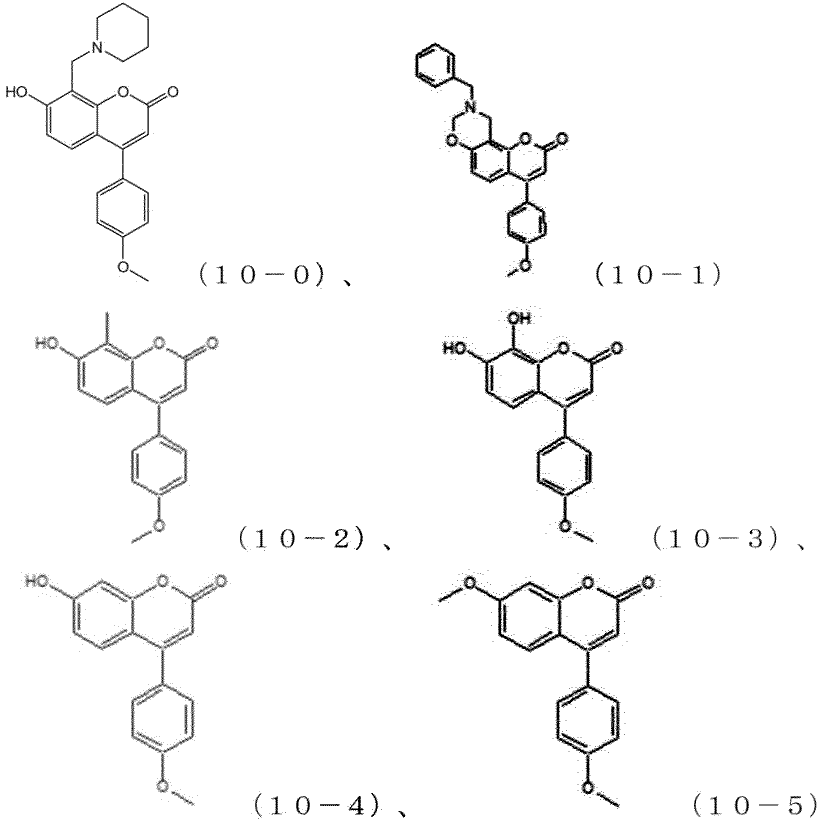

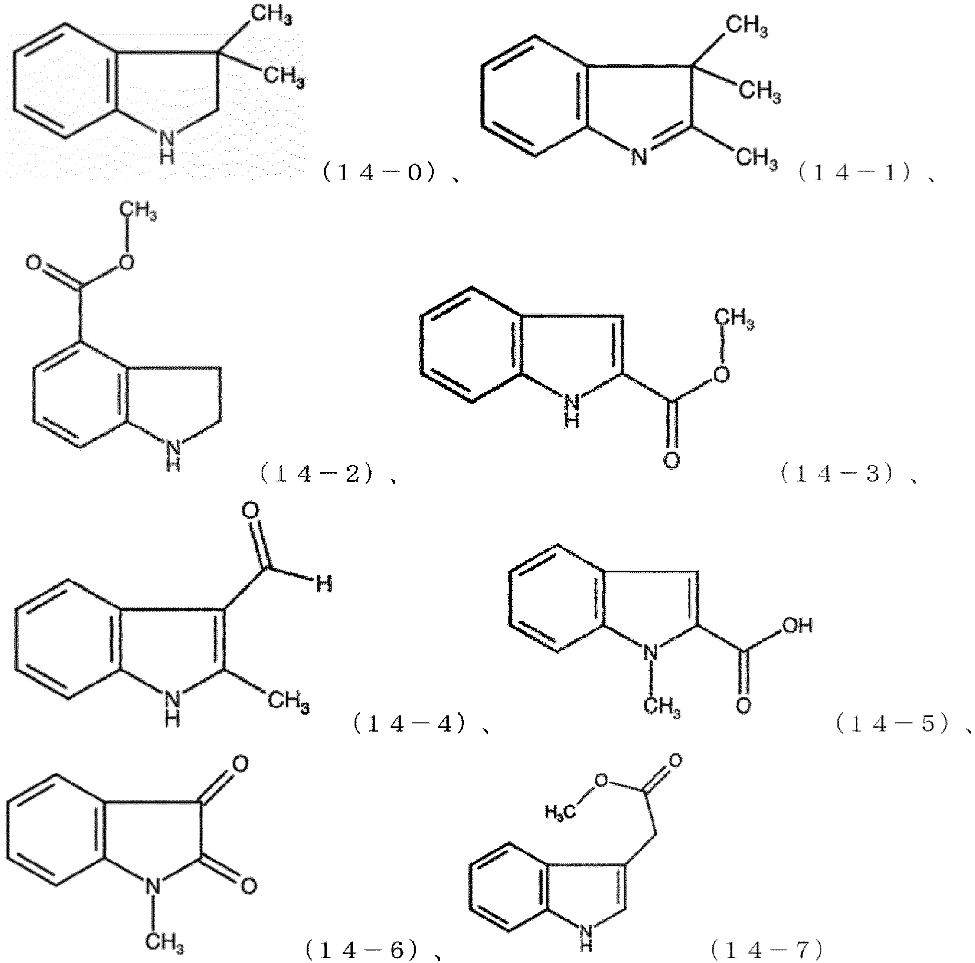

Compounds (14-0) to (14-7) are reference compounds.

The salts of compound A are pharmaceutically acceptable salts, which may be acid addition salts or salts with bases, depending on the type of substituent. Specifically, the examples of acid addition salts or salts with bases include acid addition salts with inorganic acids such as hydrochloric acid, hydrobromic acid, hydroiodic acid, sulfuric acid, nitric acid, and phosphoric acid, and organic acids such as formic acid, acetic acid, propionic acid, oxalic acid, malonic acid, succinic acid, fumaric acid, maleic acid, lactic acid, malic acid, mandelic acid, tartaric acid, dibenzoyl tartrate, ditoloyl tartrate, citric acid, methanesulfonic acid, ethanesulfonic acid, benzenesulfonic acid, p-toluenesulfonic acid, aspartic acid, and glutamic acid, salts with inorganic bases such as sodium, potassium, magnesium, calcium, and aluminum, and organic bases such as methylamine, ethylamine, ethanolamine, lysine, and ornithine, salts with various amino acids and amino acid derivatives such as acetylleucine, and ammonium salts, etc.

Furthermore, the active ingredients used in the present invention also include various hydrates, solvates, and crystalline polymorphs of compound A and salts thereof. The present invention also includes compounds labeled with various radioactive or non-radioactive isotopes.

Prostate cancer, which is the therapeutic target of the pharmaceutical composition of the present invention, is cancer that develops in the prostate gland and an antiandrogenic agent may be administered as hormone therapy. The type of prostate cancer to be treated is, but not limited to, androgen-independent prostate cancer (CRPC) that has acquired resistance to hormone therapy, which is a particularly effective target. As shown in the Examples described later, the pharmaceutical composition of the present invention can be effectively used for CRPC that has acquired resistance to hormone therapy, since the pharmaceutical composition shows remarkable anticancer effects on AR-positive CRPC and AR-negative CRPC. However, the pharmaceutical composition can also be used to treat prostate cancer that is not hormone therapy-resistant or CRPC by other mechanisms.

The inventors of the present invention have found that RNA-binding protein PSF (PTB-associated Splicing Factor) regulates factors involved in the malignant transformation of cancer in prostate cancer or breast cancer that has acquired resistance to hormone therapy. For example, PSF upregulates the expression of AR or its spliced variant, AR-V7, which is increased in Castration-resistant prostate cancer (CRPC). PSF expression is also upregulated in breast cancer OHTR cells that have acquired resistance to hormone therapy compared with the parental MCF7 cells that is sensitive to hormone therapy.

Compound A used in the present invention can bind to PSF. As shown in the Examples, the expression of AR, AR-V7, and SchLaP1, which are target genes of PSF at the RNA level, can be inhibited in 22Rv1 cells, a model cell for hormone-resistant prostate cancer. In addition, in OHT-TamR cells, a hormone-resistant model of breast cancer, the expression of ERα and SCFD2, which are downstream genes of PSF, can be inhibited.

That is to say, it is considered that compound A is effective for the treatment of hormone therapy-resistant prostate cancer by binding to PSF and suppressing the function of PSF.

A pharmaceutical composition containing one or more of the above-mentioned compound A, or salts thereof as an active ingredient can be prepared by using excipients commonly used in the art, that is to say, excipients for pharmaceutical compositions and drug carriers, etc., by a method commonly used in the art.

Administration may be oral in pill, capsule, granule, powder, or liquid form, etc., or parenteral in injection such as intra-articular, intravenous, or intramuscular, suppository, eye drop, eye ointment, transdermal solution, ointment, transdermal patch, transmucosal solution, transmucosal patch, inhalation, etc.

The examples of solid composition for oral administration include tablets, dispersions, and granules. In such solid compositions, one or more active ingredients are mixed with at least one inert excipient, such as lactose, mannitol, glucose, hydroxypropyl cellulose, microcrystalline cellulose, starch, polyvinylpyrrolidone, and/or magnesium metasilicate. The composition may contain inert additives, such as lubricants e.g., magnesium stearate, disintegrants e.g., sodium carboxymethylstatinate, stabilizer, solubilizer, etc., according to conventional methods. Tablets or pills may be coated with a sugar coating or a film of a gastric or enteric soluble substance as needed. The examples of liquid composition for oral administration include pharmaceutically acceptable emulsion, solution, suspension, syrup, or elixir, which contain commonly used inert diluent, such as purified water or ethanol. In addition to the inert diluent, the liquid composition may contain auxiliary agents such as solubilizer, wetting agent, and suspending agent, sweetening agent, flavoring agent, aromatic agent, and preservative.

The examples of injectable drug for parenteral administration include sterile aqueous or non-aqueous solution, suspension or emulsion. The examples of aqueous solvent include, distilled water or saline solution for injection. The examples of nonaqueous solvent include, propylene glycol, polyethylene glycol or vegetable oils such as olive oil, alcohols such as ethanol, or polysorbate 80 (pharmacopeia name). Such composition may further contain isotonicity agent, preservative, wetting agent, emulsifier, dispersant, stabilizer, or solubilizer. These are sterilized, for example, by filtration through a bacteria-retaining filter, the addition of a bactericidal agent, or irradiation. These can also be produced as sterile solid composition and dissolved or suspended in sterile water or sterile injectable solvent, prior to use.

The examples of topical products include ointments, hard plasters, creams, jellies, poultices, sprays, lotions, eye drops, eye ointments, etc. The examples of topical products also include commonly used ointment bases, lotion bases, aqueous or non-aqueous liquids, suspensions, emulsions, etc. The examples of ointment or lotion bases include polyethylene glycol, propylene glycol, white vaseline, white beeswax, polyoxyethylene hardened castor oil, glycerin monostearate, stearyl alcohol, cetyl alcohol, lauromacrogol, sesquioleinic acid sorbitan, etc.

Transmucosal agents, such as inhalants and nasal spray, may be used in solid, liquid, or semi-solid form and can be manufactured according to conventionally known methods. For example, known excipients, pH adjusters, preservatives, surfactants, lubricants, stabilizers and thickeners may be added appropriately. Any suitable devices for inhalation or air delivery may be used for administration. For example, transmucosal agents may be administered using known devices such as metered dose inhalation devices or atomizers, as a compound alone or as a powder in a formulated mixture, or as a solution or suspension in combination with a pharmaceutically acceptable carrier. Dry powder inhalers and the like may be used for single or multiple doses, and dry powder or powder-containing capsules may be used. Alternatively, transmucosal agents may be administered in the form of a pressurized aerosol spray using suitable ejection agents, such as preferred gases, for example, chlorofluoroalkane, hydrofluoroalkane, or carbon dioxide, etc.

Dosage varies depending on the type of disease, symptom, age, and gender of patients. The usual dosage for oral administration is approximately 0.001 mg/kg-500 mg/kg per day for adults, which is administered once or divided into two to four doses. When administered by injection, injection is administered as a rapid intravenous infusion or intravenous drip at a dose of approximately 0.0001 mg/kg-10 mg/kg once or twice per day for adults. When administered by inhalation, a single or multiple doses of approximately 0.0001 mg/kg-10 mg/kg per adult per day is administered. When administered by transdermal drug, transdermal drug is applied once or twice per day at a dose of approximately 0.01 mg/kg-10 mg/kg per day for adults.

Compound A or salts thereof, may be used in combination with various therapeutic or prophylactic agents for diseases for which compound A or salts thereof is considered to be effective. The combination use may be simultaneous, or separate and consecutive, or at desired time intervals. The simultaneous administration formulation may be a compounding agent or a separate formulation.

### <<Compound A for use in the treatment of prostate cancer>>

The compound A may be used for the treatment of prostate cancer. That is to say, this specification discloses a compound represented by the formulae (10-0) to (10-5) or a salt thereof for use in the treatment of prostate cancer.

### <<Function>>

The mechanism by which compound A B is effective in the treatment of prostate cancer has not been analyzed in detail, but can be estimated as follows. A compound A is a derivative of 4-phenylcoumarin. The skeleton of 4-phenylcoumarin is considered to be important for binding to PSF (anticancer effect). Furthermore, the presence of an oxygen atom at the 7-position of coumarin is considered to enhance the anticancer effect.

### EXAMPLES

The present invention will now be further illustrated by the following Examples.

### <<Example 1>>

In this Example, the effects of compound A on the proliferative ability of 22Rv1, a model cell line of hormone therapy-resistant prostate cancer, and LTAD (long time androgen deprived) cells, a model of hormone therapy resistance, were examined.

Cells were seeded in a 96-well plate (3×10³ cells/well), and compound 10-0 and compound 14-0 (reference) were added at the concentration of 30 µM. After 3 days, cell proliferative ability was evaluated by MTS assay. The MTS assay was performed as follows:
The reaction was carried out using Cell titer 96 (Promega). The MTS assay is an assay to measure the number of viable cells that based on the reduction reaction of tetrazolium salt [MTS;3-(4,5-dimethylthiazol-2-yl)-5-(3-carboxymethoxyphenyl)-2-(4-sulfophenyl)-2H-tetrazolium, inner salt] to the formazan product that is a chromogenic substance. After 1 hour incubation, the absorbance is measured using a microplate reader at 490 nm to evaluate cell proliferative ability.

Furthermore, the MST assay was performed in the same manner using LTAD and VCaP cells by adding 1 µM or 10 µM of compound 10-0, 10-1, 10-3, 14-0 (reference), or 14-5 (reference).

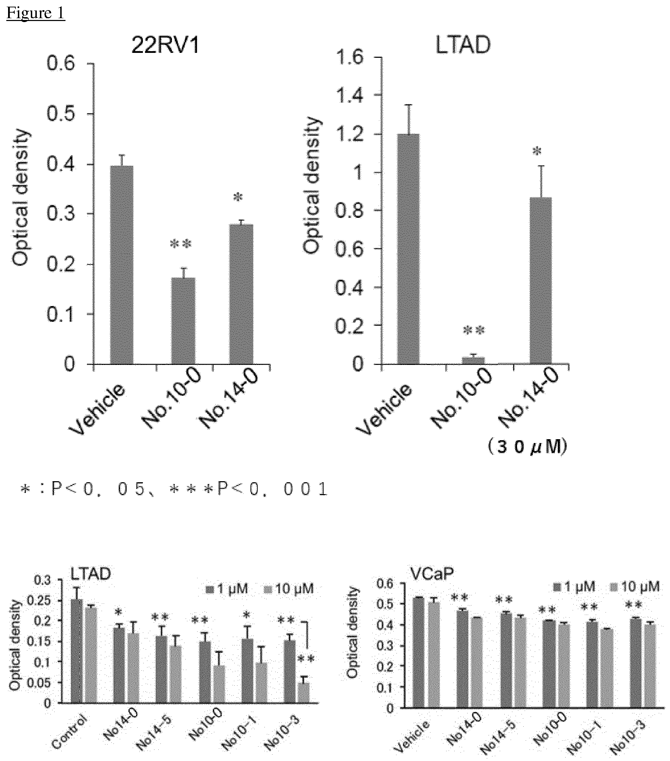

The results showed that all the compounds 10-0, 10-1, 10-3, and the reference compounds 14-0, and 14-5 significantly suppressed cell proliferation (Figure 1).

### <<Example 2>>

In this Example, the inhibitory ability of compounds 10-0, 10-1, 10-2, 10-3, 10-4, 14-0 (reference), and 14-5 (reference) to the interaction of PSF with its target RNA was examined by RNA pull-down method.

A biotin-labeled probe for CTBP1-AS was prepared using Biotin RNA Labeling Mix (Roche). The RNA probe was prepared by T7 RNA polymerase using a DNA strand in which 1.5 kb of CTBP1-AS was incorporated into T7 promoter as a template. RNA was mixed with nuclear extracts extracted from cells with RIP buffer and rotated at 4°C for 8 hours for binding. Avidin beads were added and the mixture was rotated for 2 hours to collect biotinylated RNA probes. The beads were collected by centrifugation, washed with buffer three times, and then SDS sample buffer was added to the beads, and the bound proteins were examined by Western blotting. 22Rv1 cells were used to prepare nuclear extracts in the same manner as in Example 1, except the concentrations of the added compounds were varied.

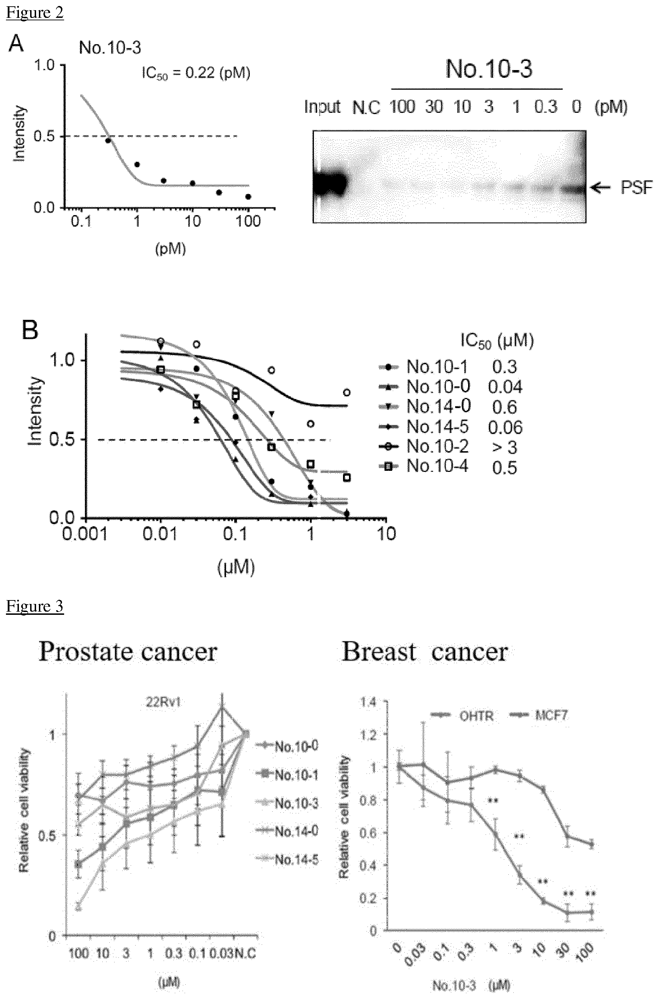

As shown in Figure 2, all compounds inhibited the binding of PSF to RNA. However, the inhibitory effect of compound 10-3 was particularly strong (IC50=0.22pM).

### <<Example 3>>

In this Example, the effects of compounds 10-0, 10-1, 10-3, 14-0, and 14-5 on cell proliferation were examined using a model cell line of hormone therapy-resistant prostate cancer 22Rv1, breast cancer cell MCF7, and a model of hormone therapy-resistant OHTR cells established from MCF7 cells. The MTS assay was performed as in Example 1.

As shown in Figure 3, each compound inhibited 22Rv1 cell proliferation. Compound 10-3 showed stronger inhibitory effect on cell proliferation of hormone therapy-resistant OHTR cells than normal breast cancer cells, MCF7.

### <<Example 4>>

In this Example, the expression levels of PSF target genes at the RNA level, AR, AR-V7, SchLaP1, and downstream signals of AR, FKBP5 and ACSL3, were examined by adding compounds 10-0, 10-1, and 10-3 to 22Rv1 cells.

22Rv1 cells were treated with compounds 10-0, 10-1, and 10-3. After 48 hours incubation, total RNA was collected to examine changes in mRNA expression of the target genes. This analysis was performed by quantitative real-time PCR as follows

Total RNA was collected from cells using ISOGEN (NIPPON Gene). cDNA was synthesized using Prime script RT reagent kit (TaKaRa Bio). Step one real-time PCR (Applied biosystem) and KAPA SYBR Fast PCR kit (NIPPON Genetics) were used to measure the mRNA expression levels of each gene and the internal control, GAPDH. The expression levels for GAPDH were calculated from the number of cycles using the ΔΔCt method.

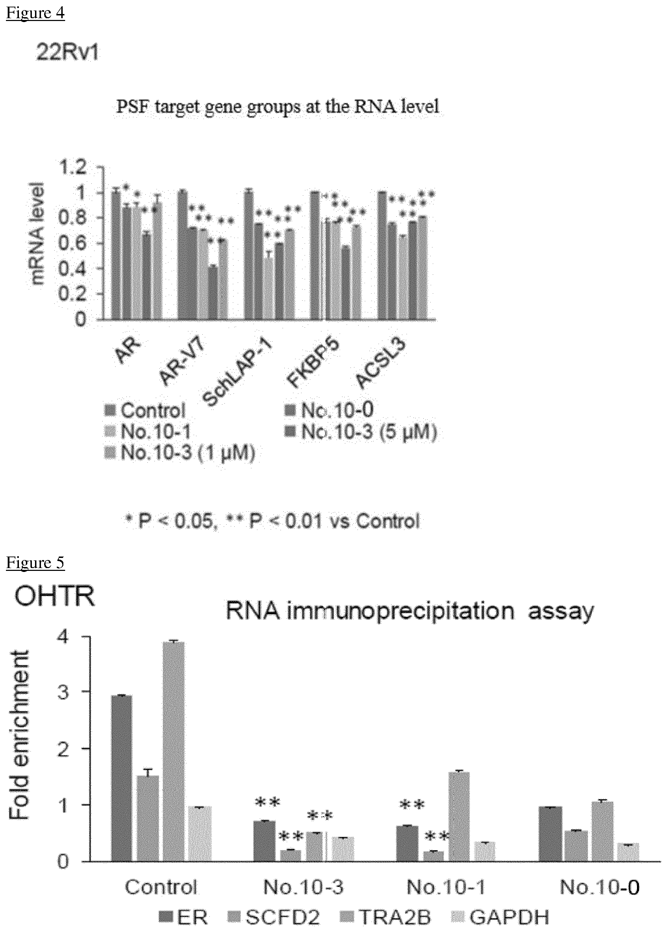

It was confirmed that the mRNA expression of AR, AR-V7, SchLaP1, FKBP5, and ACSL3 was suppressed by the administration of compound 10, 10-1, or 10-3 (10 µM) (Figure 4).

### <<Reference Example 5>>

In this Example, compounds 10-0, 10-1, and 10-3 were added to hormone therapy resistant breast cancer model OHT-TamR cells. After 48 hours of cultivation, the cells were collected, lysed, and immunoprecipitated with PSF antibody. Then total RNA binding to PSF protein was extracted. Quantitative PCR was used to quantify the amount of RNA bound to PSF for ERα, SCFD2, and TRA2B, which are target genes of PSF at the RNA level. As a control, GAPDH-bound RNA was also quantified.

OHT-TamR cells were treated with compounds 10-0, 10-1, and 10-3 for 48 hours. Total RNA binding to PSF protein was collected from immunoprecipitated beads, and the amount of each target gene and GAPDH as a control was measured. Quantitative real-time PCR was performed as in Example 4.

It was confirmed that the binding of ERα, SCFD2, and TRA2B to PSF protein was significantly inhibited by the administration of compound 10-3 (10 µM) compared to GAPDH, and binding of ERα and SCFD2 was significantly inhibited by the administration of 10-1 (Figure 5).

### <<Example 6>>

In this Example, anticancer effect of compound 10-3 in vivo was examined using a xenograft mouse model.

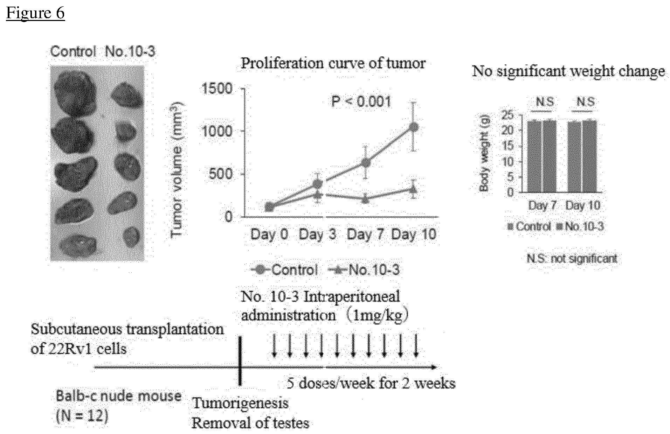

For tumor transplantation, 22Rv1 cells were adjusted to 1×10⁷, mixed with Matrigel at 1:1, and injected subcutaneously into 6-week-old male nude mice (BALB/cAJcl-nu/nu). Five to eight days after subcutaneous injection, testes were removed when the tumor volume reached about 100-200 mm³, to prepare a model of hormone therapy refractory CRPC tumors. Six mice were injected intraperitoneally with compound 10-3 at a concentration of 1 mg/kg. Vehicle (DMSO) was administered as a control. The treatment was repeated 5 times/week for 2 weeks, and the tumor diameter was measured (Figure 6).

As shown in Figure 6, tumor growth was significantly suppressed in mice treated with compound 10-3. On the other hand, administration of No.10-3 did not significantly reduce the body weight of the mice.

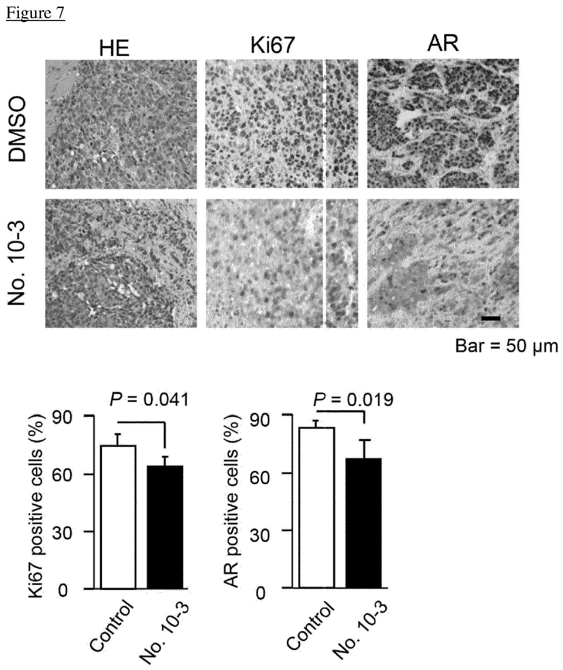

In addition, administration of compound 10-3 reduced the number of cells expressing Ki67 and AR in tumor tissues (Figure 7).

### <<Example 7>>

In this Example, the effects of compounds 10-1 and 10-3 on cell proliferation were examined using an AR-negative CRPC model DU145 cells. The MTS assay was performed as in Example 1.

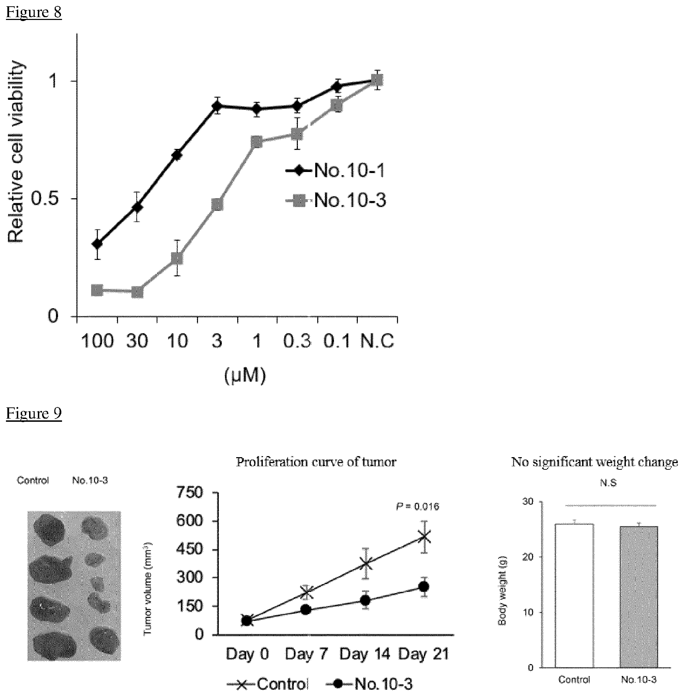

As shown in Figure 8, each compound inhibited cell proliferation in DU145 cells.

### <<Example 8>>

In this Example, anticancer effect of compound 10-3 in vivo was examined using an AR-negative CRPC tumor xenograft model, in which DU145 cells were subcutaneously transplanted to mice.

For tumor transplantation, DU145 cells were adjusted to 1×10⁷, mixed with Matrigel at 1:1, and injected subcutaneously into 6-week-old male nude mice (BALB/cAJcl-nu/nu). Five to eight weeks after subcutaneous injection, when the tumor volume reached about 100-200 mm³, compound 10-3 was injected intraperitoneally at a concentration of 5 mg/kg into 8 mice (the photo shows 4-5 mice). Controls were injected with Vehicle (DMSO). The treatment was repeated 5 times/week for 3 weeks, and the tumor diameter was measured (Figure 8).

As shown in Figure 8, tumor growth was significantly inhibited in mice treated with compound 10-3. On the other hand, administration of No.10-3 did not significantly reduce the body weight of the mice. The compounds of the present invention can inhibit the expression of AR in cancer cells, but they also show anticancer activity against AR-negative cancer cells. Therefore, it is considered that the compound exerts its anticancer effect through pathways other than suppression of AR expression.

### <<Example 9>>

In this Example, the binding site of compound 10-3 to PSF, which is the target protein of the present invention, was examined by docking assay. Furthermore, mutations were introduced into the amino acids of PSF that are predicted to interact with compound 10-3, and the inhibitory effect of compound 10-3 on the interaction of PSF with its target RNA was examined.

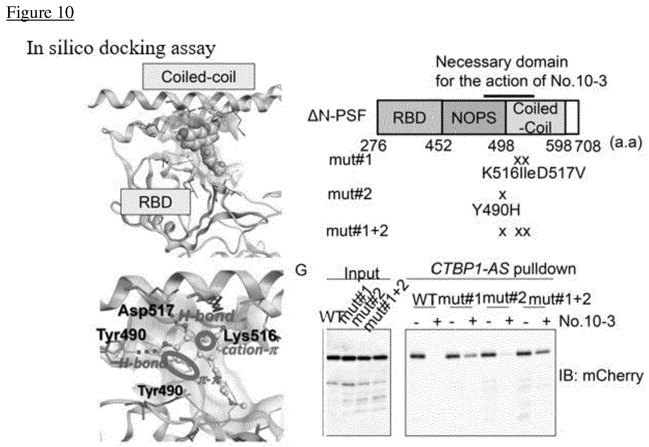

The crystal structure of human PSF (276-535 amino acids) was downloaded from the Protein Data Bank (PDB). The volume and shape of the binding cavities were analyzed using the SiteFinder module of the Molecular Operation Environment (MOE) program. As a result, compound 10-3 was estimated to bind to the Coild-Coil site from the NOPS of PSF, as shown in Figure 10.

The following combinations of mutations were introduced into the above-mentioned presumed site at position 490, tyrosine (Y), position 516, lysine (K), and position 517, aspartic acid (D).
mut#1:K516I, D517V
mut#2:Y490H
mut#1+2:Y490H, K516I, D517V

The binding of mut#1, mut#2, mut#1+2, or wild-type PSF to the RNA strand of CTBP1-AS was examined by RNA pull-down method. The inhibitory effect for binding of compound 10-3 was measured by adding compound 10-3. Compared to the wild type, mut#1, mut#2, or mut#1+2s mutant showed the decreased inhibitory effect on the PSF binding to the RNA probe by the addition of compound 10-3. Therefore, it is likely that compound 10-3 binds to tyrosine (Y) at position 490, lysine (K) at position 516, and aspartate (D) at position 517 in the region spanning from NOPS to Coild-Coil of PSF.

### INDUSTRIAL APPLICABILITY

The pharmaceutical composition of the present invention can be effectively used for the treatment of prostate cancer.

## Claims

1. A pharmaceutical composition for use in treating prostate cancer comprising:
a compound selected from the group consisting of

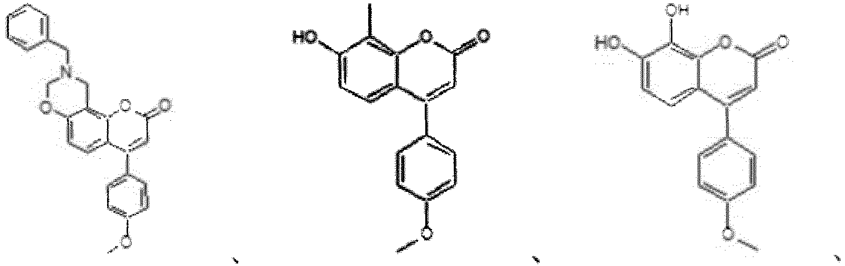

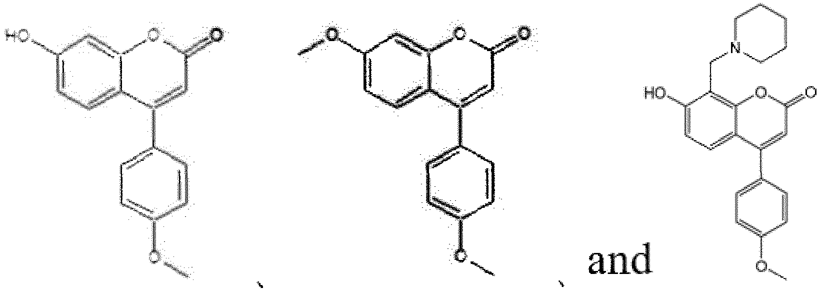

## Patentansprüche

1. Pharmazeutische Zusammensetzung zur Verwendung bei der Behandlung von Prostatakrebs, umfassend:
eine Verbindung, ausgewählt aus der Gruppe bestehend aus

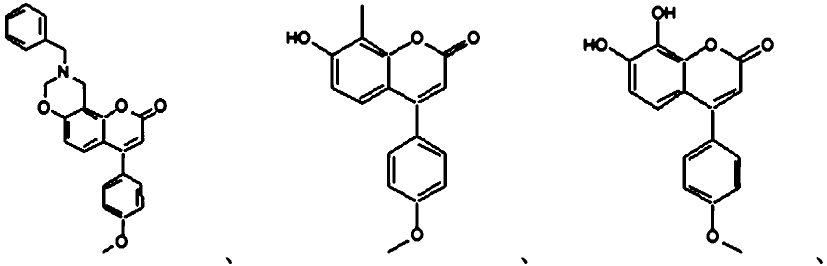

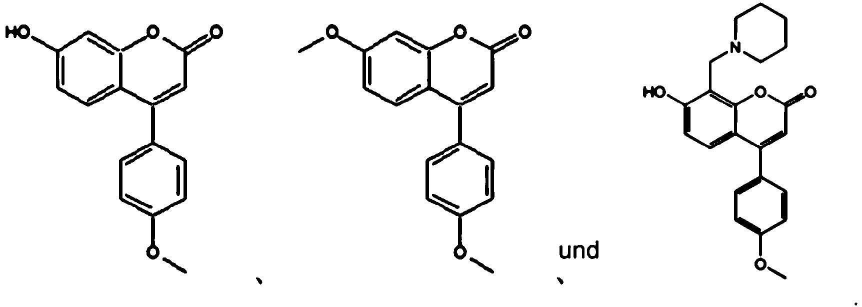

## Revendications

1. Composition pharmaceutique destinée au traitement du cancer de la prostate, comprenant :
un composé choisi dans le groupe constitué par
